# EUROPEAN PATENT SPECIFICATION

(11) **EP 3 181 142 B1**
(45) Date of publication and mention of the grant of the patent: **17.01.2018**
(21) Application number: 16205177.5
(22) Date of filing: 19.12.2016
(51) Int. Cl.: A61K 8/73, A61Q 17/00, A61Q 19/00, A61Q 19/08, A61K 8/97, A61K 8/99, A61K 36/28, A61K 36/062

(54) **TOPICAL COMPOSITIONS COMPRISING PICHIA ANOMALA AND CHICORY ROOT EXTRACTS**
TOPISCHE ZUSAMMENSETZUNGEN MIT PICHIA ANOMALA UND ZICHORIENWURZELEXTRAKTEN
COMPOSITIONS TOPIQUES COMPRENANT DU PICHIA ANOMALA ET DES EXTRAITS DE RACINES DE CHICORÉE

(30) Priority: 17.12.2015 US 201562268618 P; 12.12.2016 US 201615375365
(43) Date of publication of application: 21.06.2017
(73) Proprietor: JOHNSON & JOHNSON CONSUMER INC., Skillman, NJ 08558 (US)
(72) Inventor: BALLESTEROS, Ann Theodore, Skillman, NJ New Jersey 08558 (US); MAHMOOD, Khalid, Skillman, NJ New Jersey 08558 (US); MOREIRA, Liliam A., Skillman, NJ New Jersey 08558 (US); PARSA, Ramine, Skillman, NJ New Jersey 08558 (US); SOUTHALL, Michael D., Skillman, NJ New Jersey 08558 (US)
(74) Representative: Carpmaels & Ransford LLP

(56) References cited:
- EP-A1- 2 662 072
- WO-A2-2013/178965
- FR-A1- 2 897 266
- US-A1- 2007 141 018
- LUCIANO POLONELLIRODOLFO LORENZINIFLAVIA DE BERNARDISGIULIA MORACE: "Potential therapeutic effect of yeast killer toxin", MYCOPATHOLOGIA, KLUWER ACADEMIC PUBLISHERS, XX, vol. 96, no. 2, 1 November 1986 (1986-11-01), pages 103-107, XP009193593, ISSN: 0301-486X, DOI: 10.1007/BF00436668
- STREET R A ET AL: "Cichorium intybus: Traditional uses, phytochemistry, pharmacology, and toxicology", EVIDENCE-BASED COMPLEMENTARY AND ALTERNATIVE MEDICINE 2013 OXFORD UNIVERSITY PRESS GBR, vol. 2013, 2013, XP55160986, ISSN: 1741-427X

## Description

### Field of the Invention

The present invention provides a method of treating skin by topically applying to skin a combination of an extract of *Pichia anomala* and an extract of chicory root. Additionally, a topical composition comprising a combination of an extract of *Pichia anomala* and an extract of chicory root is provided.

### Background of the Invention

Human skin is composed of two compartments, namely a surface compartment, the epidermis, and a deep compartment, the dermis. Human skin is subject to certain aging processes.

The epidermis is in contact with the external environment. Its role consists in protecting the body from dehydration and external attacks, whether chemical, mechanical, physical or infectious. The natural human epidermis is composed mainly of three types of cells: keratinocytes, which form the vast majority, melanocytes and Langerhans' cells. Each of these cell types contributes by its specific functions to the essential role played by the skin in the body.

The dermis provides the epidermis with a solid support. It is also its source of nutrients. It is mainly composed of fibroblasts and of an extracellular matrix. It also comprises leukocytes, mast cells or tissue macrophages. It is also traversed by blood vessels and nerve fibers.

The extracellular matrix of the dermis is composed of proteins belonging to several main families: collagens, matrix glycoproteins other than collagens (fibronectin, laminin), elastin, proteoglycans and glycosaminoglycans (GAGs) in the free form (that is to say, not bonded to a protein), including hyaluronic acid. The GAG's are predominantly synthesized by fibroblasts and it is known that the skin ageing process brings about a decline in these metabolic activities, resulting in a decrease in the GAG's of the extracellular matrix of the dermis, and a decrease in cell growth, resulting in a detrimental change in the mechanical properties of the skin, in particular its firmness, its elasticity, its tonicity and/or its suppleness.

Aged skin has been shown to be characterized by reduced levels of hyaluronic acid. Hyaluronic acid is found in young skin at the periphery of collagen and elastin fibers and where these types of fibers intersect. In aged skin, such connections with hyaluronic acid disappear. The decreases in hyaluronic acid levels, which contribute to its disassociation with collagen and elastin as well as reduced water binding, may be involved in the changes noted in aged skin, including wrinkling, altered elasticity, reduced turgidity and diminished capacity to support the microvasculature of the skin. As one of the primary GAGs, hyaluronic acid can bind 1000 times its weight in water, and may help the skin retain and maintain water. It is found in all connective tissue and is produced mainly by fibroblasts and keratinocytes in the skin. Hyaluronic acid is localized not only in the dermis but also in the epidermal intercellular spaces, especially the middle spinous layer, but not in the stratum corneum (SC) or stratum granulosum. Aged skin, which is less plump than youthful skin, is characterized by decreased levels of hyaluronic acid.

The main clinical signs of skin aging are in particular the appearance of fine lines and wrinkles, which increase with age. These wrinkles can be deep, moderate or superficial, and they affect in particular the nasolabial folds, the periorbital region, the outline of the lips and the forehead (glabellar lines); these wrinkles and fine lines are reflected by a depression or folds at the surface of the skin.

Different methods have been proposed for combating wrinkles and fine lines, including the use of skin care products comprising cosmetic active agents (antiwrinkle, moisturizing, firming or tightening active agents, in particular). To this end, hyaluronic acid has been proposed as active agent capable of being used in numerous cosmetic applications, in particular as anti-ageing active agent. It also plays an important role in the moisturizing and elasticity of the skin.

However, it has been found that hyaluronic acid has a limited lifetime on the skin but very particularly when it is injected. This is because hyaluronidases are enzymes present in the skin which decompose hyaluronic acid and thus reduce the impact thereof in cosmetic compositions. The decomposition of hyaluronic acid takes place by virtue of the combined action of three different hyaluronidases. The half-life of hyaluronic acid, due to the very rapid catabolism of the molecule, varies from one tissue to another; by way of indication, it is approximately one day at the dermis and epidermis. Moreover, penetration of exogenous hyaluronic acid into the skin has proved difficult to accomplish by topical application. Delivery of exogenous hyaluronic acid by injection is used successfully, however, as a temporary dermal filling agent in soft tissue augmentation procedures.

*Pichia* is a genus of yeasts in the family Saccharomycetaceae. More than 100 species of this genus are known. The most well-known species include *Pichia anomala, Pichia guilliermondii, Pichia norvegensis,* and *Pichia ohmeri.*

*Pichia anomala* (formerly named *Hansenula anomala*) can be found in raw milk and cheese. The extracts of yeasts of the genus *Pichia* are rich in mannans, polysaccharides composed of mannose monomers. *Pichia anomala* and mannans are known to be used in the treatment of aging skin. See, for example, FR 2938768, FR 2906719, FR 2897266 and FR 2976490.

PRO-LIPISKIN® is a commercially available cosmetic ingredient containing extract of *Pichia anomala.* It is produced by Pichia strain isolated from sugar cane. It is available from Silab-France.

Common chicory, *Cichorium intybus,* is a somewhat woody, perennial herbaceous plant. Chicory is extensively cultivated for its use for fodder and food. Its above-ground parts are consumed in salads.

Root chicory (*Cichorium intybus* L.) has been cultivated in Europe as a coffee substitute. The roots are baked, ground, and used as a coffee substitute and additive. Chicory root extract is a dietary supplement or food additive produced by mixing dried, ground chicory root with water, and removing the insoluble fraction by filtration and centrifugation. Other methods may be used to remove pigments and sugars. It is used as a source of soluble fiber, specifically inulin. Fresh chicory root may contain between 13 and 23% inulin, by total weight. Chicory is also conventionally used for its choleretic, cholagogue, diuretic, depurative and digestive medicinal qualities.

Chicory extracts have further been used in cosmetic products, in particular for a pigmenting effect as described in EP-1,707,191 or EP-2,277,502, an anti-inflammatory effect as described in EP-1,962,875, an anti-radical effect for preventing cutaneous aging as described in FR-2,626,469, or in a mixture with other plants in thinning or softening cosmetic products.

US 2013/0237496 discloses the cosmetic use of a *Cichorium intybus* root hydrolyzate comprising oligofructosans as an active ingredient in a composition with cutaneous application, for acting in a way similar to vitamin D on the cells of the skin, in particular for stimulating the signaling paths regulated by the vitamin D receptor in the cutaneous cells.

VEREDINE®SP is an aqueous solution containing 5.50% chicory root extract commercially available as a cosmetic ingredient from Silab-France.

US 8,652,532 discloses an orally ingestible food composition or cosmetic composition containing glucosamine generated from plant materials through a drying process. The plant material may be root of chicory. The cosmetic composition may be topically administered and may be used for retarding the aging process of skin.

Although the art provides topical uses for extracts of *Pichia anomala* and extracts of chicory root, applicants have now discovered that topical application of a combination of these two extracts is beneficial to the skin by synergistically enhancing its production of hyaluronic acid from within, thus providing significant and unexpected benefits for skin, including improving, reducing, inhibiting, or delaying the appearance of at least one sign of aging in skin. The combination may also enhance skin barrier protection and skin moisturization. Accordingly, new methods of treating signs of skin aging, for example, are now available.

### Summary of the Invention

The present invention relates to a topical composition comprising an extract of *Pichia anomala* and an extract of chicory root, as well as cosmetic, or non-therapeutic methods of treating a sign of skin aging, comprising topically applying to skin in need of treatment for skin aging a topical composition comprising an extract of *Pichia anomala* and an extract of chicory root, wherein the extract of chicory root contains up to 20 % by weight of glucosamine and wherein the composition comprises greater than 2 weight percent of the extract of *Pichia anomala.*

The invention also relates to a cosmetic, or non-therapeutic method of treating a sign of skin aging, comprising topically applying to skin in need of treatment for skin aging a topical composition comprising an extract of Pichia anomala and an extract of chicory root, wherein
the extract of chicory root contains up to 20 % by weight of glucosamine and wherein the composition comprises greater than 2 weight percent of the extract of *Pichia anomala.*

The invention further provides a cosmetic, or non-therapeutic method of improving skin barrier function and moisturization, comprising topically applying to skin in need of improving skin barrier function and moisturization a topical composition comprising an extract of *Pichia anomala* and an extract of chicory root, wherein the extract of chicory root contains up to 20 % by weight of glucosamine and wherein the composition comprises greater than 2 weight percent of the extract of *Pichia anomala.*

### Detailed Description

The topical composition of the present invention improves the production of hyaluronic acid in the skin by synergistic action of extracts of *Pichia anomala* and chicory root.

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs. Unless otherwise indicated, percentages used to express amounts of ingredients are percentage by weight (i.e., % (W/W). Similarly weight ratios used to express relative proportions of ingredients are also determined using percentage by weight (i.e., weight ratios are calculated by dividing the percentage by weight of one ingredient by another). Unless stated otherwise, all ranges are inclusive of the endpoints, e.g., "from 4 to 9" includes the endpoints 4 and 9.

As used herein, a "product" is optionally in finished packaged form. In one embodiment, the package is a container such as a plastic, metal or glass tube or jar containing the composition. The product may further contain additional packaging such as a plastic or cardboard box for storing such container. In one embodiment, the product comprises a composition of the invention and contains instructions directing the user to apply the composition to the skin or hair.

As used herein, "topically applying" means directly laying on or spreading on outer skin, the scalp, or hair, e.g., by use of the hands or an applicator such as a wipe, roller, or spray.

As used herein, "cosmetic" refers to a beautifying substance or preparation which preserves, restores, bestows, simulates, or enhances the appearance of bodily beauty or appears to enhance the beauty or youthfulness, specifically as it relates to the appearance of tissue or skin.

As used herein, "cosmetically acceptable" means that the ingredients the term describes are suitable for use in contact with tissues (e.g., the skin or hair) without undue toxicity, incompatibility, instability, irritation, allergic response, or the like.

In certain embodiments, the compositions of the present invention are suitable for treating signs of skin aging. As used herein, "signs of skin aging" includes the presence of lines and wrinkles, loss of elasticity, uneven skin, and blotchiness. In a particularly preferred embodiment, the sign of aging is the presence of lines and wrinkles and/or loss of elasticity.

As used herein, "treating signs of skin aging" refers to mitigating, reducing, preventing, improving, or eliminating the presence or signs of skin aging described above.

As used herein, "wrinkle" includes fine lines, fine wrinkles, or coarse wrinkles. Examples of wrinkles include, but are not limited to, fine lines around the eyes (e.g., "crow's feet"), forehead and cheek wrinkles, frown-lines, and laugh-lines around the mouth.

As used herein, "loss of elasticity" includes loss of elasticity or structural integrity of the skin or tissue, including but not limited to sagging, lax and loose tissue. The loss of elasticity or tissue structure integrity may be a result of a number of factors, including but not limited to disease, aging, hormonal changes, mechanical trauma, environmental damage, or the result of an application of products, such as a cosmetics or pharmaceuticals, to the tissue.

As used herein, "uneven skin" means a condition of the skin associated with diffuse or mottled pigmentation, which may be classified as hyperpigmentation, such as post-inflammatory hyperpigmentation.

As used herein, "blotchiness" means a condition of the skin associated with redness or erythema.

As used herein, "improving the firmness of skin" means the enhancing of the firmness or elasticity of the skin, preventing the loss of firmness or elasticity of skin, or preventing or treating sagging, lax and loose skin. The firmness or elasticity of the skin can be measured by use of a cutometer. See Handbook Of Non-Invasive Methods And The Skin, eds. J. Serup, G. Jemec & G. Grove, Chapter 66.1 (2006). The loss of skin elasticity or firmness may be a result of a number of factors, including but not limited to aging, environmental damage, or the result of an application of a cosmetic to the skin.

As used herein, "improving the texture of skin" means the smoothing of the surface of the skin to remove either bumps or crevasses on the skin surface.

As used herein, "improving the appearance of wrinkles in skin" means preventing, retarding, arresting, or reversing the process of wrinkle and fine line formation in skin.

As used herein, the term "safe and effective amount" means an amount sufficient to induce the desired effect, but low enough to avoid serious side effects. The safe and effective amount of the compound, extract, or composition will vary with, e.g., the age, health and environmental exposure of the end user, the duration and nature of the treatment, the specific extract, ingredient, or composition employed, the particular carrier utilized, and like factors.

As used herein, "skin in need of improving skin barrier function and moisturization" means skin that is, but not limited to, lacking in moisture, lacking in sebum, cracked, dry, itchy, scaly, xerodermic, dehydrated, lacks suppleness, lacks radiance, dull, or lacks lipids.

As described herein, applicants have discovered that topical application of a combination of an extract of *Pichia anomala* and an extract of chicory root provides unexpectedly good skin barrier function, skin moisturization, skin anti-aging, and skin lightening benefits.

Applicants have also discovered in particular that topical application of a composition containing a combination of extracts of *Pichia anomala* and chicory root enhance the endogenous hyaluronic acid ("HA") levels in skin, providing improvements in the appearance of at least one sign of skin aging. Topical use of such a composition can increase the levels of hyaluronic acid to a direction found in younger skin thereby providing the structural support to skin to reduce the appearance of signs of aging in skin.

The topical compositions of the present invention may be used in cosmetic, or non-therapeutic, methods for treating a sign of skin aging.

The topical compositions of the present invention may also be used in cosmetic, or non-therapeutic, methods for improving skin barrier function and moisturization.

### Pichia anomala

The topical composition comprises one or more extracts of *Pichia anomala.* In particular, such extracts may be extracts produced using one of the various strains of *Pichia anomala* isolated from the fruit or other aerial parts of a plant. Any cosmetically acceptable extract of *Pichia anomala* may be used.

One example of a suitable extract of *Pichia anomala* is PRO-LIPISKIN, commercially available from Silab-France. It is produced from a strain of *Pichia anomala* present on sugar cane.

Another example of a suitable extract of *Pichia anomala* is produced from a strain of *Pichia anomala* present on fruit or leaves of Kiwi plant.

Both of the foregoing may be provided as aqueous solutions containing dry matter in the range of about 20%, more specifically 2 to 10%, most specifically 3 to 7%.

### Chicory Root

The topical composition also contains one or more extracts of chicory root (*Cichorium intybus* L.). A variety of extracts of chicory root are commercially available, however not any cosmetically acceptable extract may be used in the invention.

In a preferred embodiment, the extract of chicory root contains glucosamine in an amount of up to about 20 %, or about 8 to about 12 %, by weight based on the total weight of the extract. Such glucosamine is not separately added; it is a component of the extract of chicory root.

A suitable extract of chicory root that may be used contains about 8 to 12 % by weight glucosamine and is prepared as described in US 8652532. Specifically as described in US 8652532, chicory root is harvested, cut and dried in an oven or industrial drier at a temperature below 110° C, preferably between 80 and 105° C, most preferably 92° C or below for less than one week, preferably between 5 and 50 hours. It is also preferably to cut the chicory root into slices or cubes, preferably having a maximum width of 5 cm. The dried material is then extracted with water or other conventional solvent to make the extract of chicory root.

### Amounts

Any suitable amount of the extract of chicory root and greater than 2 weight percent of the extract of *Pichia anomala* are used in the compositions of the present invention. Preferably, the compositions comprise safe and effective amounts of the extracts. In particular, the amounts of *Pichia anomala* and chicory root extracts to be used are cosmetically acceptable and are selected to achieve the desired treatment of skin for a particular condition, as one skilled in the cosmetic art well understands.

In certain preferred embodiments, the compositions comprise from 0.5 to 20% of *Pichia anomala* extract and more preferably 0.5 to 5% of *Pichia anomala* extract. Additionally, in certain preferred embodiments, the compositions comprise from about 0.01 to 2, more preferably 0.01 to 0.2 of chicory root extract. In certain embodiments, the compositions comprise a combined amount of 0.51 to 22%, more preferably 0.7 to 5.2%, by weight of *Pichia anomala* extract and chicory root extract.

### Determination of Ceramide Profile by High-Performance Thin-layer Chromatography

The efficacy of the composition containing extracts of *Pichia anomala* and chicory root in improving skin barrier function may be measured by an increase in ceramide levels in the skin. Accordingly, in one embodiment of the present invention, the amounts of extract of *Pichia anomala* and extract of chicory root used in a composition of the invention are those effective to achieve an increase in the ceramide levels by at least 1% or higher, preferably about 5% or higher, and more preferably about 10% or higher according to the test Determination of Ceramide Profile by High-Performance Thin-layer Chromatography.

The Determination of Ceramide Profile by High-Performance Thin-layer Chromatography is performed as follows.

### Sample Extraction and Condensation

Skin equivalents or 0.5-1x10⁶ cells are homogenized with 2mL chloroform:methanol (2:1) and transferred to a vial containing 1mL Phosphate-Buffered Saline Solution. Homogenizer is rinsed with 2 2mL portions of chloroform:methanol (2:1) and the rinses are added to the vial containing the extracts and the PBS. The mixture is vortexed and the phases are allowed to separate. The organic phase is evaporated to dryness under vacuum.

### High-Performance Thin-Layer Chromatography

The residue is dissolved in 200 µL chloroform:methanol (2:1). Twenty microliters and 40 µL of sample solution are applied on the HPTLC plate (Whatman Partisil) using CAMAG Automatic TLC Sampler 4 and separated using the following sequential development system: (1) dichloromethane:ethyl acetate:acetone (80:16:4), (2) chloroform:methanol:acetone (76:16:8), and (3) hexane:chloroform:acetic acid:acetone:methanol (6:80:0.1:10:4). The plates are stained with 3% copper acetate in 8% phosphoric acid and charred at 160°C.

### Quantification

Samples are applied in parallel for positional corrections and compared to a similarly prepared blank extract (tape strip without exposure to skin lipids). Quantification is performed against known quantities of Ceramide III standard (Cosmoferm) by densitometry (CAMAG).

### Hyaluronic acid (HA) Secretion Test

In another embodiment, the efficacy of the combination of *Pichia anomala* and chicory root extracts in improving skin barrier function and / or improving the appearance of at least one sign of aging in skin may be measured by an increase in hyaluronic acid secretion. Accordingly, the combination of *Pichia anomala* and chicory root extracts used in a composition of the invention is effective for providing an increase of hyaluronic acid secretion greater than that provided by merely adding the increases in hyaluronic acid produced by each extract alone. In one embodiment, the composition of the invention provides at least a 1.5 fold, or, more preferably, at least a 2.0 fold, increase in hyaluronic acid secretion over the additive fold increase when measured in accordance with the Hyaluronic acid (HA) Secretion test as follows.

The Hyaluronic acid (HA) Secretion test is performed as follows.

Human dermal fibroblasts are maintained in flask in growth medium consisting of Dulbeccos' Modified Eagle Medium (DMEM) plus 10% fetal bovine serum, 50 units/ml penicillin and 50µg/ml streptomycin. Cells are seeded at 20,000 cells per well in a 96 well plate. After 24 hours incubation, cells are treated with test compositions dissolved in DMSO or DMSO without extracts (as control) prepared in DMEM+2%FBS. Culture media is collected at 48 hours post-treatment, and measured for HA (Hyaluronic acid) secretion using Hyaluronan ELISA kit (Echelon, cat. #K-1200) following the manufacturer protocol. To assess activity, the colorimetric chance is measured at 405 nm and the results expressed as a fold change over untreated controls.

The fold increase provided by the combination is calculated as the fold change in HA secreted from application of the combined extracts divided by the sum of: a) the fold change in HA secreted from of *Pichia anomala* alone and b) the fold change in HA secreted from of extract of chicory root alone.

### Topical Compositions

The compositions of the present invention are applied topically to human skin or hair. Accordingly, the composition may further include a cosmetically acceptable topical carrier that may be from about 50% to about 99.99%, by weight, of the composition (e.g., from about 80% to about 99%, by weight, of the composition). In a preferred embodiment of the invention, the cosmetically acceptable topical carrier includes water.

The compositions may be made into a wide variety of product types that include but are not limited to lotions, creams, gels, sticks, sprays, ointments, cleansing liquid washes and solid bars, shampoos and hair conditioners, hair fixers, pastes, foams, powders, mousses, shaving creams, wipes, patches, hydrogels, film-forming products, facial masks and skin masks, films and make-up such as foundations, and mascaras. These product types may contain several types of cosmetically acceptable topical carriers including, but not limited to solutions, suspensions, emulsions such as microemulsions and nanoemulsions, gels, solids and liposomes. The following are non-limiting examples of such carriers. Other carriers can be formulated by those of ordinary skill in the art.

The compositions useful in the present invention can be formulated as solutions. Solutions typically include an aqueous or organic solvent (e.g., from about 50% to about 99.99% or from about 90% to about 99% of a cosmetically acceptable aqueous or organic solvent). Examples of suitable organic solvents include propylene glycol, polyethylene glycol, polypropylene glycol, glycerol, 1,2,4-butanetriol, sorbitol esters, 1,2,6-hexanetriol, ethanol, and mixtures thereof.

Compositions useful in the subject invention may be formulated as a solution comprising an emollient. Such compositions preferably contain from about 2% to about 50% of an emollient(s). As used herein, "emollients" refer to materials used for the prevention or relief of dryness, such as by preventing the transepidermal loss of water from the skin. Examples of emollients include those known in the art. Examples of particularly suitable emollients include vegetable oils, mineral oils, fatty esters, and the like.

A lotion can be made from such a solution. Lotions typically contain from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically contains from about 5% to about 50% (e.g., from about 10% to about 20%) of an emollient(s) and from about 45% to about 85% (e.g., from about 50% to about 75%) of water.

The composition of the present invention may include water or alternatively be anhydrous or be an ointment that includes no water but organic and/or silicone solvents, oils, lipids and waxes. An ointment may contain a simple base of animal or vegetable oils or semi-solid hydrocarbons. An ointment may contain from about 2% to about 10% of an emollient(s) plus from about 0.1% to about 2% of a thickening agent(s).

The composition may be formulated as an emulsion. If the topical carrier is an emulsion, from about 1% to about 10% (e.g., from about 2% to about 5%) of the topical carrier contains an emulsifier(s). Emulsifiers may be nonionic, anionic or cationic. Examples of emulsifiers are well known in the art.

Lotions and creams can be formulated as emulsions. Typically such lotions contain from 0.5% to about 5% of an emulsifier(s). Such creams typically contain from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type or the oil-in-water-in-oil type, are also useful in the subject invention. In general, such single or multiphase emulsions contain water, emollients, and emulsifiers as essential ingredients.

The compositions of this invention can also be formulated as a gel (e.g., an aqueous, alcohol, alcohol/water, or oil gel using a suitable gelling agent(s)). Suitable gelling agents for aqueous and/or alcoholic gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically contains between about 0.1% and 5%, by weight, of such gelling agents.

The compositions of the present invention can also be formulated into a solid formulation (e.g., a wax-based stick, soap bar composition, powder, or a wipe containing powder).

The compositions useful in the subject invention may contain, in addition to the aforementioned components, a wide variety of additional oil-soluble materials and/or water-soluble materials conventionally used in compositions for use on skin and hair, at their art-established levels.

### Additional Cosmetically Active Agents

The compositions of the present invention may further comprise any of a variety of additional cosmetically active agents. Examples of suitable additional active agents include: skin lightening agents, darkening agents, additional anti-aging agents, tropoelastin promoters, collagen promoters, anti-acne agents, shine control agents, anti-microbial agents such as anti-yeast agents, anti-fungal, and anti-bacterial agents, anti-inflammatory agents, anti-parasite agents, external analgesics, sunscreens, photoprotectors, antioxidants, keratolytic agents, detergents/surfactants, moisturizers, nutrients, vitamins, energy enhancers, anti-perspiration agents, astringents, deodorants, hair removers, hair growth enhancing agents, hair growth delaying agents, firming agents, hydration boosters, efficacy boosters, anti-callous agents, agents for skin conditioning, anti-cellulite agents, odor-control agents such as odor masking or pH-changing agents, and the like.

Examples of various suitable additional cosmetically acceptable actives include hydroxy acids; benzoyl peroxide; D-panthenol; UV filters such as but not limited to avobenzone (PARSOL 1789), bisdisulizole disodium (NEO HELIOPAN AP), diethylamino hydroxybenzoyl hexyl benzoate (UVINUL A Plus), ecamsule (MEXORYL SX), methyl anthranilate, 4-aminobenzoic acid (PABA), cinoxate, ethylhexyl triazone (UVINULT 150), homosalate, 4-methylbenzylidene camphor (PARSOL 5000), octyl methoxycinnamate (Octinoxate), octyl salicylate (Octisalate), padimate O (ESCALOL 507), phenylbenzimidazole sulfonic acid (ENSULIZOLE), polysilicone-15 (PARSOL SLX), trolamine salicylate, Bemotrizinol (TINOSORB S), benzophenones 1-12, dioxybenzone, drometrizole trisiloxane (MEXORYL XL), iscotrizinol (UVASORB HEB), octocrylene, oxybenzone (EUSOLEX 4360), sulisobenzone, bisoctrizole (TINOSORB M), titanium dioxide, zinc oxide; carotenoids; free radical scavengers; spin traps; retinoids and retinoid precursors such as retinol, retinoic acid and retinyl palmitate; ceramides; polyunsaturated fatty acids; essential fatty acids; enzymes; enzyme inhibitors; minerals; hormones such as estrogens; steroids such as hydrocortisone; 2-dimethylaminoethanol; copper salts such as copper chloride; peptides containing copper, coenzyme Q10; amino acids such a proline; vitamins; lactobionic acid; acetyl-coenzyme A; niacin; riboflavin; thiamin; ribose; electron transporters such as NADH and FADH2; and other botanical extracts such as oat, aloe vera, Feverfew, Soy, Shiitake mushroom extracts, and derivatives and mixtures thereof.

In certain preferred embodiments, the compositions comprise a combination of *Pichia anomala* and chicory root extracts and at least one additional skin moisturizing active agent. In certain preferred embodiments, the skin care compositions comprise the combination of *Pichia anomala* and chicory root extracts and at least one additional agent for improving the appearance of at least one sign of aging in skin. Examples of suitable additional agents improving the appearance of at least one sign of aging in skin include, but are not limited to, tropoelastin promoters, collagen promoters, retinoids, hyaluronic acid including cross-linked hyaluronic acid, dimethylaminoethanol, N,N,N',N'-tetrakis(2-hydroxypropyl)ethylenediamine, alpha hydroxy acids, polyhydroxyacids, and combinations of two or more thereof.

"Tropoelastin promoters," as used herein, refers to a class of compounds that possess the biological activity of enhancing the production of tropoelastin. Tropoelastin promoters, according to the present invention, include all natural or synthetic compounds that are capable of enhancing the production of tropoelastin in the human body.

Examples of suitable tropoelastin promoters include, but are not limited to, blackberry extracts, cotinus extracts, feverfew extracts, and bimetal complexes having copper and/or zinc constituents. The bimetal complex having copper and/or zinc constituents may be, for example, copper-zinc citrate, copper-zinc oxalate, copper-zinc tartarate, copper-zinc malate, copper-zinc succinate, copper-zinc malonate, copper-zinc maleate, copper-zinc aspartate, copper-zinc glutamate, copper-zinc glutarate, copper-zinc fumarate, copper-zinc glucarate, copper-zinc polyacrylic acid, copper-zinc adipate, copper-zinc pimelate, copper-zinc suberate, copper-zinc azealate, copper-zinc sebacate, copper-zinc dodecanoate, or combinations thereof. In a preferred embodiment, the tropoelastin promoter is selected from blackberry extracts, cotinus extracts, feverfew extracts, and combinations thereof. In a particularly preferred embodiment, the tropoelastin promoter is selected from blackberry extracts, feverfew extracts, and combinations thereof.

By "blackberry extract," it is meant a blend of compounds isolated from the plant of the genus *Rubus,* and preferably *Rubus fruticosus.* In one embodiment, the compounds are isolated from the flowers of the plant. In a further embodiment, the compounds are isolated from dried flowers of the plant. Such compounds may be isolated from one or more part of the plant (*e.g*., the whole plant, flower, seed, root, rhizome, stem, fruit and/or leaf of the plant). In a preferred embodiment, the blackberry extract is a blackberry leaf extract. One particularly suitable blackberry extract is produced by extracting the leaves of *Rubus fruticosus* with a mixture of water and ethanol compounded to an activity of about 5% to about 10%, with a maltodextrin matrix, commercially available from Symrise Inc. of Teterboro, NJ, and is sold under the name SYMMATRIX.

Compositions of the present invention may include a cosmetically effective amount of one or more tropoelastin promoters such as those described above. The compositions preferably include, on an active basis, from about 0.1% to about 10% of the tropoelastin promoters, more preferably from about 0.5% to about 5% of tropoelastin promoters, and most preferably from about 0.5% to about 2% of the tropoelastin promoters.

"Collagen promoter," as used herein, refers to compounds that possess the biological activity of enhancing the production of collagen. "Non-retinoid collagen promoters" according to the present invention include all natural or synthetic compounds that are not retinoids, or derived from retinoids, and are capable of enhancing the production of collagen in the human body.

Examples of suitable collagen promoters include, but are not limited to the following: Retinoids including retinol, retinaldehyde, and retinoic acid, extracts of feverfew (*Tanacetum parthenium*), extracts of *Centella asiatica,* and extracts of *Siegesbeckia orientalis*; extracts of soy; collagen-promoting peptides; ursolic acid; and asiaticoside.
*Centella asiatica,* also known as *Violette marronne* on Reunion Island, Gotu Kola or Indian pennywort in India, *Centella repanda* in North America, and Talapetraka in Madagascar, is a polymorphous herb and belongs to the family of *Umbelliferae* (*Apiaceae*), particularly to the *Hydrocotyle* subfamily. It grows wild throughout the tropics and prefers moist and shady regions at an altitude of about 600 to 1200 meters above sea level. *Centella asiatica* has three varieties: Typica, Abyssinica, and Floridana. The herb is known and used for its healing, sedative, analgesic, antidepressant, antiviral and antimicrobial properties. The biological activity of the herb appears to be due to the presence of triterpene molecules in the herb. A suitable extract of *Centella asiatica* is available as TECA from Bayer Consumer HealthCare of Basel, Switzerland.

By "extracts of *Siegesbeckia orientalis,"* is meant any of various extracts of the plant *Siegesbeckia orientalis,* including Darutoside available from Sederma (Croda International Group of Edison, NJ).

Suitable collagen-promoting peptides include the following matrikine peptides, (*i.e.,* a peptide derived from the degradation of extracellular matrix proteins - collagen, elastin, or proteoglycan) including palmitoyl pentapeptides, such as MATRIXYL from Sederma (Croda International Group of Edison, NJ); GHK copper peptide available as PROCYTE from Photomedex of Montgomeryville, PA;Palmitoyl GHK peptide available as Biopoeptide CL from Sederma (Croda International Group of Edison, NJ); Biomimetic tetrapeptides, such as those available as Chronoline Tri Peptide from Unipex of Quebec, Canada ; and Palmitoyl tri-peptide, available as Syn-Coll from DSM of Basel, Switzerland.

Ursolic acid is also known as pentacyclic triterpene acid, Prunol, Malol, Urson, beta-ursolic acid and 3-Beta-Hydroxy-Urs-12-En-28-Oic Acid. It is commercially available for example from Sigma-Aldrich of St. Louis, MO.

Asiaticoside, also known chemically as: [6-[[3,4-dihydroxy-6-(hydroxymethyl)-5-(3,4,5-trihydroxy-6-methyloxan-2-yl)oxyoxan-2-yl]oxymethyl]-3,4,5-trihydroxyoxan-2-yl] 10,11-dihydroxy-9-(hydroxymethyl)-1,2,6a,6b,9,12a-hexamethyl-2,3,4,5,6,6a,7,8,8a,10,11,12,13,14b-tetradecahydro-1H-picene-4a-carboxylate) is commercially available for example from Bayer Santé Familiale Division Serdex, 69, Boulevard Victor Hugo 93400 SAINT-OUEN France.

Compositions of the present invention may include a cosmetically effective amount of one or more collagen promoters. The compositions preferably include, on an active basis, from about 0.1% to about 10% of the collagen promoters, more preferably from about 0.5% to about 5% of collagen promoters, and most preferably from about 0.5% to about 2% of the collagen promoters.

The compositions of the present invention may comprise additionally at least one skin lightening active agent. Examples of suitable skin lightening active agents include, but are not limited to, tyrosinase inhibitors, melanin-degradation agents, melanosome transfer inhibiting agents including PAR-2 antagonists, exfoliants, sunscreens, retinoids, antioxidants, Tranexamic acid, tranexamic acid cetyl ester hydrochloride, skin bleaching agents, linoleic acid, adenosine monophosphate disodium salt, Chamomilla extract, allantoin, opacifiers, talcs and silicas, zinc salts, and the like, and other agents as described in Solano et al. Pigment Cell Res. 19 (550-571) and Ando et al. Int J Mol Sci 11 (2566-2575).

Examples of suitable tyrosinase inhibitors include but, are not limited to, Vitamin C and its derivatives, Vitamin E and its derivatives, Kojic Acid, Arbutin, resorcinols, hydroquinone, Flavones e.g. Licorice flavanoids, Licorice root extract, Mulberry root extract, Dioscorea Coposita root extract, Saxifraga extract and the like, Ellagic acid, Salicylates and derivatives, Glucosamine and derivatives, Fullerene, Hinokitiol, Dioic acid, Acetyl glucosamine, 5,5'-dipropyl-biphenyl-2,2'-diol (Magnolignan), 4-(4-hydroxyphenyl)-2-butanol (4-HPB), combinations of two or more thereof, and the like. Examples of vitamin C derivatives include, but are not limited to, ascorbic acid and salts, Ascorbic Acid-2-Glucoside, sodium ascorbyl phosphate, magnesium ascorbyl phosphate, and natural extract enriched in vitamin C. Examples of vitamin E derivatives include, but are not limited to, alpha-tocopherol, beta, tocopherol, gamma-tocopherol, delta-tocopherol, alpha-tocotrienol, beta-tocotrienol, gamma-tocotrienol, delta-tocotrienol and mixtures thereof, tocopherol acetate, tocopherol phosphate and natural extracts enriched in vitamin E derivatives. Examples of resorcinol derivatives include, but are not limited to, resorcinol, 4-substituted resorcinols like 4-alkylresorcinols such as 4-butyresorcinol (rucinol), 4-hexylresorcinol (Synovea HR, Sytheon), phenylethyl resorcinol (Symwhite, Symrise), 1-(2,4-dihydroxyphenyl)-3-(2,4-dimethoxy-3-methylphenyl)-Propane (nivitol, Unigen) and the like and natural extracts enriched in resorcinols. Examples of salicylates include, but are not limited to, 4-methoxy potassium salicylate, salicylic acid, acetylsalicylic acid, 4-methoxysalicylic acid and their salts. In certain preferred embodiments, the tyrosinase inhibitors include a 4-substituted resorcinol, a vitamin C derivative, or a vitamin E derivative. In more preferred embodiments, the tyrosinase inhibitor comprises Phenylethyl resorcinol, 4-hexyl resorcinol, or ascorbyl-2-glucoside.

Examples of suitable melanin-degradation agents include, but are not limited to, peroxides and enzymes such as peroxidases and ligninases. In certain preferred embodiments, the melanin-inhibiting agents include a peroxide or a ligninase.

Examples of suitable melanosome transfer inhibiting agents including PAR-2 antagonists such as soy trypsin inhibitor or Bowman-Birk Inhibitor, Vitamin B3 and derivatives such as Niacinamide, Essential soy, Whole Soy, Soy extract. In certain preferred embodiments, the melanosome transfer inhibiting agents includes a soy extract or niacinamide.

Examples of exfoliants include, but are not limited to, alpha-hydroxy acids such as lactic acid, glycolic acid, malic acid, tartaric acid, citric acid, or any combination of any of the foregoing, beta-hydroxy acids such as salicylic acid, polyhydroxy acids such as lactobionic acid and gluconic acid, and mechanical exfoliation such as microdermabrasion. In certain preferred embodiments, the exfoliants include glycolic acid or salicylic acid.

Examples of retinoids include, but are not limited to, retinol (Vitamin A alcohol), retinal (Vitamin A aldehyde), retinyl acetate, retinyl propionate, retinyl linoleate, retinoic acid, retinyl palmitate, isotretinoin, tazarotene, bexarotene, Adapalene, combinations of two or more thereof and the like. In certain preferred embodiments, the retinoid is selected from the group consisting of retinol, retinal, retinyl acetate, retinyl propionate, retinyl linoleate, and combinations of two or more thereof. In certain more preferred embodiments, the retinoid is retinol.

Examples of antioxidants include, but are not limited to, water-soluble antioxidants such as sulfhydryl compounds and their derivatives (*e.g*., sodium metabisulfite and N-acetylcysteine, glutathione), lipoic acid and dihydrolipoic acid, stilbenoids such as resveratrol and derivatives, lactoferrin, iron and copper chelators and ascorbic acid and ascorbic acid derivatives (*e.g*., ascobyl-2-glucoside, ascorbyl palmitate and ascorbyl polypeptide). Oil-soluble antioxidants suitable for use in the compositions of this invention include, but are not limited to, butylated hydroxytoluene, retinoids (*e.g*., retinol and retinyl palmitate), tocopherols (*e.g*., tocopherol acetate), tocotrienols, and ubiquinones. Natural extracts containing antioxidants suitable for use in the compositions of this invention, include, but not limited to, extracts containing flavonoids and isoflavonoids and their derivatives (*e.g*., genistein and diadzein), extracts containing resveratrol and the like. Examples of such natural extracts include grape seed, green tea, black tea, white tea, pine bark, feverfew, parthenolide-free feverfew, oat extracts, blackberry extract, cotinus extract, soy extract, pomelo extract, wheat germ extract, Hesperedin, Grape extract, Portulaca extract, Licochalcone, chalcone, 2,2'-dihydroxy chalcone, Primula extract, propolis, and the like.

The additional cosmetically active agent may be present in a composition in any suitable amount, for example, in an amount of from about 0.0001% to about 20% by weight of the composition, e.g., about 0.001% to about 10% such as about 0.01% to about 5%. In certain preferred embodiments, in an amount of 0.1% to 5% and in other preferred embodiments from 1% to 2%.

Compositions of the present invention may include a cosmetically effective amount of one or more anti-inflammatory compounds.

Examples of suitable anti-inflammatory agents include substituted resorcinols, (E)-3-(4-methylphenylsulfonyl)-2-propenenitrile (such as "Bay 11-7082," commercially available from Sigma-Aldrich of St. Louis, Missouri), tetrahydrocurcuminoids (such as Tetrahydrocurcuminoid CG, available from Sabinsa Corporation of Piscataway, NJ), extracts and materials derived from the following: *Phellodendron amurense* Cortex Extract (PCE), Non-Denatured Soy (*Glycine max*), Feverfew (*Tanacetum parthenium*), Ginger (*Zingiber officinale*), Ginko (*Ginkgo biloba*), Madecassoside (*Centella asiatica* extract ingredient), Cotinus (*Cotinus coggygria*), Butterbur Extract (*Petasites hybridus*), Goji Berry (*Lycium barbarum*), Milk Thistle Extract (*Silybum marianum*), Honeysuckle (*Lonicera japonica*), Basalm of Peru (*Myroxylon pereirae*), Sage (*Salvia officinalis*), Cranberry Extract (*Vaccinium oxycoccos*)*,* Amaranth Oil (*Amaranthus cruentus*), Pomegranate (*Punica granatum*), Yerbe Mate (*Ilex paraguariensis* Leaf Extract), White Lily Flower Extract (*Lilium candidum*), Olive Leaf Extract (*Olea europaea*), Phloretin (apple extract), Oat Flour (*Aveena sativa*), Lifenol (Hops: *Humulus lupulus*) Extract, Bugrane P (*Ononis spinosa*)*,* Licochalcone (Licorice: *Glycyrrhiza inflate* extract ingredient), Symrelief (Bisabolol and Ginger extract), combinations of two or more thereof, and the like.

In one embodiment, the anti-inflammatory agent is a resorcinol. Particularly suitable substituted resorcinols include 4-hexyl resorcinol and 4-octylresorcinol, particularly 4-hexyl resorcinol. 4-Hexyl resorcinol is commercially available as SYNOVEA HR from Sytheon of Lincoln Park, NJ. 4-Octylresorcinol is commercially available from City Chemical LLC of West Haven, Connecticut.

By "extracts of feverfew," it is meant extracts of the plant "*Tanacetum parthenium*," such as may be produced according to the details set for the in US Patent Application Publication No. 2007/0196523, entitled "PARTHENOLIDE FREE BIOACTIVE INGREDIENTS FROM FEVERFEW (TANACETUM PARTHENIUM) AND PROCESSES FOR THEIR PRODUCTION." One particularly suitable feverfew extract is commercially available as about 20% active feverfew, from Integrated Botanical Technologies of Ossining, NY.

In the skin care composition of the invention, the ratio of the amounts of the combined *Pichia anomala* and chicory root extracts to the anti-inflammatory compound may be varied. For example, the extract and the anti-inflammatory compound may be present in a weight ratio (which is determined by dividing the amount by weight of the dry extract by the amount by weight of the anti-inflammatory compound) of about 0.001 to about 100, preferably about 0.01 to about 10, more preferably about 0.25 to about 2.

A variety of other materials may also be present in the compositions of the present invention. In certain preferred embodiments, the composition comprises one or more topical ingredients selected from the group consisting of: surfactants, chelating agents, emollients, humectants, conditioners, preservatives, opacifiers, fragrances and the like.

What is meant by an emollient is a compound that helps to maintain the soft, smooth, and pliable appearance of the skin (*e.g*., by remaining on the skin surface or in the stratum corneum to act as a lubricant). Examples of suitable emollients include those found in Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY), and include, but are not limited to, petrolatum, hexyldecyl stearate and plant, nut, and vegetable oils such as macadamia nut oil, rice bran oil, grape seed oil, palm oil, prim rose oil, hydrogenates peanut oil, and avocado oil.

What is meant by a humectant is a compound intended to increase the water content of the top layers of skin (*e.g*., hygroscopic compounds). Examples of suitable humectants include those found Chapter 35, pages 399-415 (Skin Feel Agents, by G Zocchi) in Handbook of Cosmetic Science and Technology (edited by A. Barel, M. Paye and H. Maibach, Published in 2001 by Marcel Dekker, Inc New York, NY) and include, but are not limited to, glycerin, sorbitol or trehalose (*e.g*., α,α- trehalose, β,β-trehalose, α,β-trehalose) or a salt or ester thereof (*e.g*., trehalose 6-phosphate).

In one embodiment, the composition contains glycerin. For example, the composition contains at least 10 wt % glycerin. The composition may contain at least 12 wt % glycerin.

In another embodiment, the composition has a pH of 6.5 or less. For example the composition may have a pH of 5.5 or less.

In a particular embodiment, the composition contains at least 10 wt% glycerin and has a pH or 6.5 or less. Such a composition provides substantial increases in hyaluronic acid production when used to treat skin according to the invention.

What is meant by a surfactant is a surface-active agent intended to cleanse or emulsify. Examples of suitable surfactants include those found in Chapter 37, pages 431-450 (Classification of surfactants, by L. Oldenhove de Guertechin) in Handbook of Cosmetic Science and Technology (edited by A. Barel, M. Paye and H. Maibach, Published in 2001 by Marcel Dekker, Inc New York, NY) and include, but are not limited to anionic surfactants such as sulfates, cationic surfactants such as betaines, amphoteric surfactants such as sodium coco glycinate, noionic surfactants such as alkyl polyglucosides.

Examples of suitable chelating agents include those which are capable of protecting and preserving the compositions of this invention. Preferably, the chelating agent is ethylenediamine tetracetic acid ("EDTA"), and more preferably is tetrasodium EDTA, available commercially from Dow Chemical Company of Midland, Michigan under the trade name VERSENE 100XL.

Suitable preservatives include, for example, parabens, quaternary ammonium species, phenoxyethanol, benzoates, DMDM hydantoin, organic acids and are present in the composition in an amount, based upon the total weight of the composition, from about 0 to about 1 percent or from about 0.05 percent to about 0.5 percent.

Any of a variety of conditioners which impart additional attributes, such as gloss to the hair, are suitable for use in this invention. Examples include, but are not limited to, volatile silicone conditioning agent having an atmospheric pressure boiling point less than about 220°C. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Michigan under the tradename, "DC-345" and mixtures thereof, and preferably include cyclomethicone fluids. Other suitable conditioners include cationic polymers, including polyquarterniums, cationic guar, and the like.

Any of a variety of commercially available pearlescent or opacifying agents are suitable for use in the composition. Examples of suitable pearlescent or opacifying agents include, but are not limited to, mono or diesters of (a) fatty acids having from about 16 to about 22 carbon atoms and (b) either ethylene or propylene glycol; mono or diesters of (a) fatty acids having from about 16 to about 22 carbon atoms (b) a polyalkylene glycol of the formula: HO-(JO)ₐ-H, wherein J is an alkylene group having from about 2 to about 3 carbon atoms; and a is 2 or 3; fatty alcohols containing from about 16 to about 22 carbon atoms; fatty esters of the formula: KCOOCH₂L, wherein K and L independently contain from about 15 to about 21 carbon atoms; inorganic solids insoluble in the shampoo composition, and mixtures thereof.

Any fragrance compositions suitable for use on skin may be used in accord with the present invention.

In certain preferred embodiments, the present invention is in the form of a substrate comprising a composition of the present invention. Any suitable substrate may be used. Examples of suitable substrates and substrate materials are disclosed, for example, in U.S. Published Application Nos. 2005/0226834 and 2009/0241242.

In certain preferred embodiments, the substrate is a wipe, glove, or a facial mask. Preferably, such embodiments comprise a water-insoluble substrate as such is defined in the cited references above. For certain embodiments, the water-insoluble substrate may have a size and shape such that it covers the face of a human user to facilitate placing the water-insoluble substrate about the face of the user as a mask substrate. For example, the water-insoluble mask substrate may have openings for a mouth, nose, and/or eyes of the user. Alternatively, the water-insoluble substrate may have no such openings. Such a configuration without openings may be useful for embodiments of the invention in which the water-insoluble substrate is intended to be draped over a non-facial expanse of skin or if the water-insoluble substrate is intended to be used as wipe. The water-insoluble substrate may have various shapes, such as an angular shape (*e.g*., rectangular) or an arcuate shape such as circular or oval. For certain embodiments, the substrate is a glove such as described in U.S. Published Application No 2006/0141014. In one embodiment of the invention, the product includes a plurality of water-insoluble substrates of different shapes.

The present invention further comprises a cosmetic, or non-therapeutic method of improving the barrier function and moisturization of skin by applying to skin in need of improving skin barrier function and moisturization an extract of combined *Pichia anomala* and chicory root extracts. The method comprises for example topically applying a composition of the present invention comprising combined *Pichia anomala* and chicory root extracts to skin in need of improving skin barrier function and moisturization. Such topical application may be to any skin in need of treatment on the body, for example skin of the face, lips, neck, chest, back, arms, axilla, hands, feet and/or legs. The combined *Pichia anomala* and chicory root extracts are preferably applied in an effective amount that results in the desired improvement of skin barrier function being achieved.

The present invention further comprises a cosmetic, or non-therapeutic method of improving the appearance of at least one sign of skin aging by applying to skin in need of improving the appearance of at least one sign of skin aging combined *Pichia anomala* and chicory root extracts. The method comprises for example topically applying a composition of the present invention comprising combined *Pichia anomala* and chicory root extracts to skin in need of treatment of at least one sign of skin aging. Such topical application may be to any skin in need of treatment on the body, for example skin of the face, lips, neck, chest, back, arms, axilla, hands, feet and/or legs. The combined *Pichia anomala* and chicory root extracts are preferably applied in an effective
amount that results in the desired improvement in the appearance of at least one sign of skin aging being achieved.

Any suitable method of applying the composition to the skin in need may be used. For example, the composition may be applied directly from a package to the skin in need, by hand to the skin in need, or may be transferred from a substrate such as a wipe or mask, or a combination of two or more thereof. In other embodiments, the composition may be applied via a dropper, tube, roller, spray, and patch or added to a bath or otherwise to water to be applied to the skin, and the like. The composition may be applied in a variety of manners /forms, including, without limitation, as a leave-on cream, mask, and / or serum.

In certain embodiments, the methods of the present invention comprise applying at least two different compositions or products comprising *Pichia anomala* or chicory root extracts to the skin. For example, the methods may comprise applying a first composition comprising an extract of *Pichia anomala,* followed by applying a second composition comprising an extract of chicory root that is different from the first composition, to the skin in need of treatment.

In certain preferred embodiments, the first and second composition may be independently selected from the group consisting of lotions, cleansers, masks, wipes, creams, serums, gels, and the like. In certain preferred embodiments, at least one of the first and second compositions is a cleanser, lotion, cream, essence, or serum and the other is a facial mask or wipe. In certain other preferred embodiments, at least one of the first and second compositions is a cleanser and the other is a lotion or cream.

The composition and formulations and products containing such compositions of the present invention may be prepared using methodology that is well known by an artisan of ordinary skill. These compositions may be useful in treating skins of aging such as wrinkles, loss of elasticity, uneven skin including reducing blotchiness. The composition may be used on a routine basis and is substantially free of skin irritants.

The following non-limiting examples further illustrate the present invention.

### Example 1

Hyaluronic acid production in human dermal fibroblasts after treating the cells with extracts from *Pichia anomala* and/or chicory root were determined using the Hyaluronic acid (HA) Secretion test described above. The results are shown in Tables 1 and 2, in which values represent average of independent studies.

The extracts of *Pichia anomala* were supplied in liquid form treated as 100% stock solution, and directly added to the culture medium in the appropriate amount to reach the final tested concentrations.

The extract of chicory root was a powder containing approximately 12% by weight glucosamine prepared as described in US Patent No. 8652532. It was re-suspended in Phosphate Buffer Saline (PBS) to make a 10% (weight/volume) stock solution. This stock solution was then further diluted in the cell culture medium to reach the final tested concentrations.

The two extracts were added to the culture media either alone or together at the described doses and their resulting effects on HA secretion were used to determine fold change of HA production compared with untreated.

**Table 1**

| *Pichia anomala* extract* (wt%) | Fold change of HA production over untreated | | |
|---|---|---|---|
| | + Chicory Root Extract** (wt%) | | |
| | 0.0% | 0.05% | 0.2% |
| 0.0 | 1.00 (Untreated) | 0.9 | 1.06 |
| 1 | 1.13 | 2.68 | 1.97 |
| 2.5 | 1.02 | 2.59 | 3.47 |
| **Pichia anomala* obtained from Kiwi plant | | | |
| **Chicory root extract prepared as described in US 8652532. | | | |

**Table 2**

| *Pichia anomala* extract* (wt%) | Fold change of HA production over untreated | | |
|---|---|---|---|
| | + Chicory Root Extract** (wt%) | | |
| | 0.0% | 0.05% | 0.2% |
| 0.0 | 1.00 (Untreated) | 0.9 | 1.06 |
| 1 | 0.84 | 2.38 | 2.74 |
| 2.5 | 1.06 | 2.36 | NT |
| **Pichia anomala* obtained from Sugar cane | | | |
| **Chicory root extract prepared as described in US 8652532 | | | |
| NT = Not tested | | | |

This data indicate treatment of human dermal fibroblasts with a combination of chicory root and Pichia anomala extracts resulted in an unexpected synergistic stimulation of HA production.

It may be noted that cells treated with 2.5% extract of Pichia anomala from kiwi and 0.2% chicory root extract provided a 3.47 fold change in HA production over untreated, which amounted to a 1.67 change over the additive fold changes.

### Example 2

To further study the effects over a wider range of concentrations, extracts of *Pichia anomala* from Kiwi plant and chicory root extract prepared as described in US Patent No. 8652532 were selected. Following the same protocols from Example 1, the two extracts were added to the culture media either alone or together at the described doses and their resulting effects on HA secretion was used to determine fold change of HA production compared with untreated. The results are shown in Table 3. The fold changes from Table 3 were used to determine synergy effects over additive effects for each combination using the formula stated above. The synergy effects over additive effects are reported in Table 4.

**Table 3**

| *Pichia anomala* extract* (wt%) | Fold change of HA production over untreated | | | | |
|---|---|---|---|---|---|
| | + Chicory Root Extract** (wt%) | | | | |
| | 0.0 | 0.01% | 0.02% | 0.05% | 0.2% |
| 0.0 | 1.00 (Untreated) | 0.83 | 0.87 | 0.77 | 0.7 |
| 0.5 | 0.79 | 0.66 | 0.77 | 0.84 | 1.65 |
| 1 | 0.81 | 0.77 | 1.0 | 1.32 | 4.36 |
| 5 | 2.32 | 10.26 | 16.09 | 79.66 | 25.7 |
| **Pichia anomala* obtained from Kiwi plant | | | | | |
| **Chicory root extract prepared as described in US patent 8652532. | | | | | |

**Table 4**

| *Pichia anomala* extract* (wt%) | Fold change of HA production over additive (P. anomala extract + Chicory root extract) | | | |
|---|---|---|---|---|
| | + Chicory Root Extract** (wt%) | | | |
| | 0.01% | 0.02% | 0.05% | 0.2% |
| 0.5 | <1.00 | <1.00 | <1.00 | 1.1 |
| 1 | <1.00 | <1.00 | <1.00 | 2.87 |
| 5 | 3.26 | 5.04 | 25.77 | 8.48 |
| **Pichia anomala* obtained from Kiwi plant | | | | |
| **Chicory root extract prepared as described in US patent #8652532. | | | | |

The results in Table 3 and 4 clearly show treatment of human dermal fibroblasts with a combination of extracts of *Pichia anomala* and chicory root resulted in an unexpected, synergistic stimulation of HA production. The effects were observed in a dose-dependent manner at doses of 0.5-5 wt% of extract of *Pichia anomala* in combination with doses of chicory root extract ranging from 0.01 to 0.2 wt% yielding a synergy effects ranging from 1.1 to 25.77 fold. Thus, it is expected that using the combination of *Pichia anomala* and chicory root extracts at select ratios would provide superior anti-aging activity.

Data presented in Tables 1-4 taken together suggest that significant synergy effects over additive effects were observed when *Pichia anomala* extract was used in an amount of >2% in combination with Chicory root extract. More importantly the effective observed combinations are 5% of *Pichia anomala* extract with 0.01-0.2% of Chicory root extract resulting in a synergy effects in the range of 3.26-25.77 folds (Table 4). Use of 1 or 2.5% of *Pichia anomala* extract with 0.2% of Chicory root extract also produces significant synergy effects (Tables 1 & 4).

### Example 3

A combination of: 1) an ingredient comprising an extract of *Pichia Pastoris* and resveratrol (METABIOTICS commercially available from Lonza Personal Care) with 2) the chicory root extract described in Example 1 was tested for HA production in the Hyaluronic acid (HA) Secretion Test.

The results are shown in Table 5.

**Table 5**

| | Fold production of HA over untreated | Synergy effects over additive effects: >1.00 |
|---|---|---|
| Untreated 0% | 1.00 | N/A |
| *Pichia Pastoris* /resveratrol 1% | 0.75 | N/A |
| *Pichia Pastoris* /resveratrol 2.5% | 0.60 | N/A |
| *Pichia Pastoris* /resveratrol 1%+ Chicory root extract 0.05% | 1.50 | <1.00 |
| *Pichia Pastoris* /resveratrol 2.5%+ Chicory root extract 0.05% | 0.83 | <1.00 |
| *Pichia Pastoris* /resveratrol 1%+ Chicory root extract 0.2% | 0.86 | <1.00 |
| *Pichia Pastoris* /resveratrol 2.5%+ Chicory root extract 0.2% | 1.28 | <1.00 |

Use of an extract of Pichia pastoris in combination with chicory root extract failed to provide synergistic production of HA in human dermal fibroblasts. This indicates not all Pichia extracts are the same.

### Example 4

A combination of: 1) *Pichia anomala* described in Example 1, and 2) VEDERINE® SP, a chicory root extract commercially available from Silab-France, was tested for HA production in the Hyaluronic acid (HA) Secretion Test.

The results are shown in Table 6.

**Table 6**

| | Fold production of HA over untreated | Synergy effects over additive effects: >1.00 |
|---|---|---|
| Untreated 0% | 1.00 | N/A |
| VEDERINE 0.2% | 0.79 | N/A |
| VEDERINE 2% | 0.79 | N/A |
| *Pichia anomala* 1% + VEDERINE 0.2% | 0.80 | <1.00 |
| *Pichia anomala* 5% + VEDERINE 0.2% | 0.73 | <1.00 |
| *Pichia anomala* 1% + VEDERINE 2% | 1.07 | 1 |
| *Pichia anomala* 5% + VEDERINE 2% | 0.58 | <1.00 |

Use of the Pichia anomala in combination with VEDERINE® SP failed to provide synergistic production of HA in human dermal fibroblasts. Thus indicating not all chicory root extracts are the same.

### Example 5

The following four skin care compositions are made according to the invention.

**Table 7**

| INCI Name | % weight |
|---|---|
| Water | 66.59 |
| Sodium Chloride | 0.01 |
| Extract of *Pichia anomala* | 5.00 |
| Chicory Root Extract | 0.05 |
| Petrolatum | 4.00 |
| Dodecylhexadecanol | 2.50 |
| Dimethicone | 1.25 |
| Isopropyl Palmitate | 3.00 |
| Distearyldimonium Chloride | 5.00 |
| Glycerin | 12.00 |
| Benzyl Alcohol | 0.60 |

The composition shown in Table 7 above can be prepared as follows: water is added to a process vessel. Mixing is begun and salt is added and mixed until dissolved. Heat is applied and mixing continued until 85°C is reached. Glycerin is then added while mixing is continued and the temperature is maintained at 85°C. Distearyldimonium chloride is added, as is petrolatum and dodecylhexadecanol, dimethicone, and isopropyl palmitate. The composition is mixed at 85°C for another 10-15 minutes. The composition is then removed from heat and continued to mix and cooled. At 40°C, chicory root extract, extract of *Pichia anomala,* and benzyl alcohol are added, q.s. with water and continued to be mixed and cooled to 30-35°C. The composition is then filled into packaging.

**Table 8**

| INCI Name | % weight |
|---|---|
| Water | 65.35 |
| Petrolatum | 4.00 |
| Dodecylhexadecanol | 2.50 |
| Dimethicone | 1.25 |
| BHT | 0.10 |
| Isopropyl Palmitate | 3.00 |
| Distearyldimonium Chloride | 5.00 |
| Extract of *Pichia anomala* | 5.00 |
| Chicory Root Extract | 0.2 |
| Glycerin | 12.00 |
| Glycine Soja (Soybean) Oil and Retinol | 1.00 |
| Benzyl Alcohol | 0.60 |

The composition shown in Table 8 above can be prepared as follows: Water is added to a process vessel and the temperature is set to 85°C. Mixing is begun and glycerin is added and mixed until dissolved. Distearyldimonium chloride and petrolatum are added along with dodecylhexadecanol, dimethicone and isopropyl palmitate. The composition is mixed at 85°C for another 10-15 minutes. The composition is then removed from heat and Glycine Soja (Soybean) Oil and Retinol, chicory root extract, and Extract of *Pichia anomala* are added to the mix and cooled. At 40°C, benzyl alcohol is added, q.s. with water and continued to be mixed and cooled to 30-35°C. The composition is then filled into packaging.

**Table 9**

| INCI Name | % weight |
|---|---|
| Deionized Water | 77.49 |
| Pentylene glycol | 5.00 |
| Extract of *Pichia anomala* | 0.5 |
| Chicory Root Extract | 0.01 |
| Carthamus Tinctorius Oleosome | 10.00 |
| C12-15 Alkyl Benzoate | 4.00 |
| Ammonium Acryloyldimethyl-taurate/VP Copolymer | 2.00 |
| Chrysanthemum Parthenium (Feverfew) Leaf/Flower/Stem Juice | 1.00 |

The composition shown in Table 9 above can be prepared as follows: chicory root extract and Extract of *Pichia anomala* are measured and dissolved in pentylene glycol and deionized water is added to form Phase A. Carthamus Tinctorius Oleosome and C12-15 Alkyl Benzoate are mixed to form Phase B. Phase B is added to Phase A very slowly under continuous mixing. Mixing is continued for 15 minutes until a uniform emulsion is formed. Ammonium Acryloyldimethyl-taurate/VP Copolymer is added to the emulsion under continuous mixing at high speed to obtain a thick, smooth and homogenous formulation.

**Table 10**

| INCI Name | % weight |
|---|---|
| Water | 66.95 |
| Cross-linked polyacrylic acid | 0.60 |
| Disodium EDTA | 0.20 |
| Steareth-2 | 0.75 |
| Steareth-21 | 1.50 |
| C12-15 Alkyl Benzoate | 2.00 |
| Dimethicone | 5.00 |
| Phenonip XB | 1.00 |
| *Peucedanum graveolens* (10% active) | 10.00 |
| Maltodextrin, *Rubus fruticosus* (Blackberry) Leaf Extract (10%active) | 10.00 |
| Extract of *Pichia anomala* | 0.5 |
| Chicory Root Extract | 0.2 |
| Glycerin | 1.00 |

The composition shown in Table 7 above can be prepared as follows: an oil phase is prepared by adding C 12-15 alkyl benzoate to a clean glass beaker. Agitation is begun and the vessel is heated to 55-60° C. When the oil phase reaches 55°C or higher, Brij 72 and Brij 721 are added. When the oil phase reaches 55-60° C., it is held at that temperature and mixed for 15 min (or until uniform). The temperature is then held at 55-60° C. with mixing until addition to water phase. A water phase is prepared by adding water to a clean glass beaker. Agitation is begun and the vessel heated to 55-60° C. Disodium EDTA and Ultrez 10 are added. At 55-60° C., the ingredients are mixed for 15 min or until homogeneous. The temperature is then held at 55-60° C. with mixing for phasing. The oil phase is added to the water phase with increased agitation and then mixed at high speed for 10-20 min. At 50° C. or lower, dimethicone is added. At 40° C, or lower, Phenonip XB is added. The phases are then mixed for 10 min or until uniform. Sodium hydroxide is added (target pH is 5.4). The composition is then mixed for 10 min or until uniform. *Peucedanum graveolens* (10% active) and Maltodextrin, *Rubus fruticosus* (Blackberry) Leaf Extract (10% active) are then added. Chicory root extract and Extract of *Pichia anomala* are measured and dissolved in Glycerin and added to the mixture. This is mixed until uniform. Water is then added to QS and the composition is then mixed for 10 minutes.

### Example 6

A skin care composition according to the invention was made and tested for hyaluronic acid production in normal explants of human skin measured by immunohistofluorescence. The composition was compared against a placebo (containing neither extract of *Pichia anomala* nor Chicory Root Extract).

The test was performed as follows. 8 mm diameter punches were cut from human plasties and maintained in survival conditions in culture medium. The explants were treated topically every day with the test formulas. The explants were recovered, fixed, dehydrated and embedded in paraffin. 4 µm sections were then prepared with a Leica RM2125RT microtome.

Visualization was conducted with an IX 70 microscope coupled to an image analysis system. Fluorescence intensity (green) is proportional to the synthesis of hyaluronic acid. Quantitative analysis of images was conducted with software.

The results are expressed as fluorescence intensity based on the total surface of the dermis (in Arbitrary Units (AU)).

The ingredients in the composition and the results are shown in Table 11.

**Table 11**

| | |
|---|---|
| **% Upregulation of Hyaluronic Acid versus Placebo** | **34** |
| **Synthesis of Hyaluronic Acid (AU)** | **42.97** |
| | |
| **Ingredient Name** | **% w/w** |
| Purified Water | 69.59 |
| Colloidal Oatmeal | 1.00 |
| Sodium Chloride | 0.01 |
| Distearyldimonium Chloride | 5.00 |
| Cetyl Alcohol | 2.50 |
| Dimethicone (50 cst) | 1.25 |
| Petrolatum | 4.00 |
| Isopropyl Palmitate | 3.00 |
| Glycerin | 12.00 |
| Benzyl alcohol | 0.60 |
| Extract of *Pichia anomala* | 1.00 |
| Chicory Root Extract | 0.05 |
| Total | 100.00 |
| | |
| Target pH | 5.5 |

This composition, containing 12 % wt glycerin, and having a pH of 5.5, demonstrated an excellent increase in hyaluronic acid production.

## Claims

1. A topical composition comprising an extract of *Pichia anomala* and an extract of chicory root (*Cichorium intybus*), wherein the extract of chicory root contains up to 20 % by weight of glucosamine and wherein the composition comprises greater than 2 weight percent of the extract of *Pichia anomala.*

2. The topical composition of claim 1, wherein the extract of *Pichia anomala* is prepared from a strain of *Pichia anomala* present on kiwi fruit / leaves.

3. The topical composition of claim 1, wherein the extract of *Pichia anomala* is prepared from a strain of *Pichia anomala* present on sugar cane.

4. The topical composition of claim 1 or claim 2, wherein the extract of chicory root inherently contains up to 12% by weight of glucosamine.

5. The topical composition of any of the preceding claims comprising greater than 2 to 20 weight percent of the extract of *Pichia anomala.*

6. The topical composition of any of the preceding claims comprising greater than 2 to 5 weight percent of the extract of *Pichia anomala.*

7. The topical composition of any of the preceding claims comprising 0.01 to 0.2 weight percent of the extract of chicory root.

8. The topical composition of any of the preceding claims further comprising at least 10 wt % glycerin.

9. The topical composition of any of the preceding claims having a pH of 6.5 or less.

10. A cosmetic, or non-therapeutic method of treating a sign of skin aging, comprising topically applying to skin in need of treatment for skin aging a topical composition comprising an extract of *Pichia anomala* and an extract of chicory root (*Cichorium intybus*), wherein the extract of chicory root contains up to 20 % by weight of glucosamine and wherein the composition comprises greater than 2 weight percent of the extract of *Pichia anomala.*

11. A cosmetic, or non-therapeutic method of improving skin barrier function and moisturization, comprising topically applying to skin in need of improving skin barrier function and moisturization a topical composition comprising an extract of *Pichia anomala* and an extract of chicory root (*Cichorium intybus*), wherein the extract of chicory root contains up to 20 % by weight of glucosamine and wherein the composition comprises greater than 2 weight percent of the extract of *Pichia anomala.*

## Patentansprüche

1. Topische Zusammensetzung, umfassend einen *Pichia-anomala-*Extrakt und einen Zichorienwurzelextrakt (*Cichorium intybus*), wobei der Zichorienwurzelextrakt bis zu 20 Gew.-% Glucosamin enthält und wobei die Zusammensetzung mehr als 2 Gew.-% des *Pichia-anomala-*Extrakts umfasst.

2. Topische Zusammensetzung nach Anspruch 1, wobei der *Pichia-anomala*-Extrakt von einem *Pichia-anomala-*Stamm, der auf Kiwifrüchten/Blättern vorliegt, hergestellt wird.

3. Topische Zusammensetzung nach Anspruch 1, wobei der *Pichia-anomala*-Extrakt von einem *Pichia-anomala-*Stamm, der auf Zuckerrohr vorliegt, hergestellt wird.

4. Topische Zusammensetzung nach Anspruch 1 oder Anspruch 2, wobei der Zichorienwurzelextrakt von Natur aus bis zu 20 Gew.-% Glucosamin enthält.

5. Topische Zusammensetzung nach einem der vorhergehenden Ansprüche, umfassend mehr als 2 bis 20 Gew.-% des *Pichia-anomala-*Extrakts*.*

6. Topische Zusammensetzung nach einem der vorhergehenden Ansprüche, umfassend mehr als 2 bis 5 Gew.-% des *Pichia-anomala-*Extrakts*.*

7. Topische Zusammensetzung nach einem der vorhergehenden Ansprüche, umfassend 0,01 bis 0,2 Gew.-% des Zichorienwurzelextrakts.

8. Topische Zusammensetzung nach einem der vorhergehenden Ansprüche, weiterhin umfassend mindestens 10 Gew.-% Glycerin.

9. Topische Zusammensetzung nach einem der vorhergehenden Ansprüche mit einem pH-Wert von 6,5 oder weniger.

10. Kosmetisches oder nichttherapeutisches Verfahren zum Behandeln eines Anzeichens von Hautalterung, bei dem man auf Haut, die einer Behandlung für Hautalterung bedarf, topisch eine topische Zusammensetzung umfassend einen *Pichia-anomala-*Extrakt und einen Zichorienwurzelextrakt (*Cichorium intybus*) aufträgt, wobei der Zichorienwurzelextrakt bis zu 20 Gew.-% Glucosamin enthält und wobei die Zusammensetzung mehr als 2 Gew.-% des *Pichia-anomala*-Extrakts umfasst.

11. Kosmetisches oder nichttherapeutisches Verfahren zum Verbessern der Hautbarrierefunktion und Feuchthaltung der Haut, bei dem man auf Haut, die einer Verbesserung der Hautbarrierefunktion und der Feuchthaltung der Haut bedarf, topisch eine topische Zusammensetzung umfassend einen *Pichia-anomala-*Extrakt und einen Zichorienwurzelextrakt (*Cichorium intybus*) aufträgt, wobei der Zichorienwurzelextrakt bis zu 20 Gew.-% Glucosamin enthält und wobei die Zusammensetzung mehr als 2 Gew.-% des *Pichia-anomala-*Extrakts umfasst.

## Revendications

1. Composition topique comprenant un extrait de *Pichia anomala* et un extrait de racine de chicorée sauvage (*Cichorium intybus*), dans laquelle l'extrait de racine de chicorée sauvage contient jusqu'à 20% en poids de glucosamine et où la composition comprend plus de 2% en poids de l'extrait de *Pichia anomala.*

2. Composition topique selon la revendication 1, dans laquelle l'extrait de *Pichia anomala* est préparé à partir d'une souche de *Pichia anomala* présente sur les fruits/feuilles de kiwi.

3. Composition topique selon la revendication 1, dans laquelle l'extrait de *Pichia anomala* est préparé à partir d'une souche de *Pichia anomala* présente sur de la canne à sucre.

4. Composition topique selon la revendication 1 ou selon la revendication 2, dans laquelle l'extrait de racine de chicorée sauvage contient intrinsèquement jusqu'à 12% en poids de glucosamine.

5. Composition topique selon l'une quelconque des revendications précédentes, comprenant plus de 2 à 20% en poids de l'extrait de *Pichia anomala.*

6. Composition topique selon l'une quelconque des revendications précédentes, comprenant plus de 2 à 5% en poids de l'extrait de *Pichia anomala.*

7. Composition topique selon l'une quelconque des revendications précédentes, comprenant de 0,01 à 0,2% en poids de l'extrait de racine de chicorée sauvage.

8. Composition topique selon l'une quelconque des revendications précédentes, comprenant en outre au moins 10% en poids de glycérol.

9. Composition topique selon l'une quelconque des revendications précédentes, ayant un pH de 6,5 ou moins.

10. Méthode cosmétique ou non thérapeutique de traitement d'un signe de vieillissement cutané, comprenant l'application par voie topique, à une peau ayant besoin d'un traitement contre le vieillissement cutané, d'une composition topique comprenant un extrait de *Pichia anomala* et un extrait de racine de chicorée sauvage (*Cichorium intybus*), où l'extrait de racine de chicorée sauvage contient jusqu'à 20% en poids de glucosamine et où la composition comprend plus de 2% en poids de l'extrait de *Pichia anomala.*

11. Méthode cosmétique ou non thérapeutique d'amélioration de la fonction de barrière et de l'hydratation cutanées, comprenant l'application par voie topique, à une peau ayant besoin d'une amélioration de la fonction de barrière et de l'hydratation cutanées, d'une composition topique comprenant un extrait de *Pichia anomala* et un extrait de racine de chicorée sauvage (*Cichorium intybus*), où l'extrait de racine de chicorée sauvage contient jusqu'à 20% en poids de glucosamine et où la composition comprend plus de 2% en poids de l'extrait de *Pichia anomala.*
